# Europäisches Patentamt

# European Patent Office

# Office européen des brevets

(19)

(11) Veröffentlichungsnummer: **0 021 311**
**B1**

(12)

# EUROPÄISCHE PATENTSCHRIFT

(45) Veröffentlichungstag der Patentschrift:
05.05.82

(21) Anmeldenummer: 80103354.9

(22) Anmeldetag: 16.06.80

(51) Int. Cl.³: **C 12 N 9/04**

(54) Verfahren zur Gewinnung von Cholesterinoxidase.

(30) Priorität: **20.06.79 DE 2924875**

(43) Veröffentlichungstag der Anmeldung:
**07.01.81 Patentblatt 81/1**

(45) Bekanntmachung des Hinweises auf die Patenterteilung:
**05.05.82 Patentblatt 82/18**

(84) Benannte Vertragsstaaten:
**AT BE CH DE FR GB IT LI LU NL SE**

(73) Patentinhaber: **BOEHRINGER MANNHEIM GMBH,
Sandhofer Strasse 112-132 Postfach 31 01 20,
D-6800 Mannheim 31-Waldhof (DE)**

(72) Erfinder: **Gauhl, Helmgard, Kustermannstrasse 31,
D-8132 Tutzing (DE)**
Erfinder: **Schawohl, Georg, Kafkastrasse 18,
D-8000 München 83 (DE)**
Erfinder: **Seidel, Hans, Dr., Waxensteinstrasse 6,
D-8132 Tutzing (DE)**
Erfinder: **Beaucamp, Klaus, Dr.,
von-Kühlmann-Strasse 15, D-8132 Tutzing (DE)**

(74) Vertreter: **Weickmann, Heinrich, Dipl.-Ing Patentanwälte
Dipl.Ing.H.Weickmann et al, Dipl.Phys.Dr.K.Fincke
Dipl.Ing.F.A.Weickmann Dipl.Chem.B.Huber,
Dr.-Ing.H.Liska Möhlstrasse 22, D-8000 München 86 (DE)**

(56) Entgegenhaltungen:
FR-A-2 047 147
FR-A-2 330 766
JP-A-7 667 786

CHEMICAL ABSTRACTS, vol. 73, Nr. 17,
26. Oktober 1970, Seite 94,
Zusammenfassung 84 787e
Columbus Ohio, USA
ZHUKOVA, A. I. et al. "Production of cholesterol
oxidase by some micro-organisms".

(56) Entgegenhaltungen:
CHEMICAL ABSTRACTS, vol. 80, Nr. 3,
21. Januar 1974, Seite 150,
Zusammenfassung 11 702h
Columbus, Ohio, USA
FUKUDA, H. et al. "Anticholesterol
substances produced by Streptomyces. I. Method to
screen anticholesterol substances produced by
microbes and a new cholesterol oxidase produced by
Streptomyces Violascens".

(56) Entgegenhaltungen:
**CHEMICAL ABSTRACTS, vol. 85, Nr. 19,
8. November 1976, Seite 393,
Zusammenfassung 141 331a
Columbus, Ohio, USA**

**CHEMICAL ABSTRACTS, vol. 86, Nr. 11,
14. März 1977, Seite 196,
Zusammenfassung 67 327f
Columbus, Ohio, USA
NOMA, A. et al. "Comparative studies on cholesterol
oxidases from different sources".**

**CHEMICAL ABSTRACTS, vol. 87, Nr. 11,
12. September 1977, Seite 257,
Zusammenfassung 80 812a
Columbus, Ohio, USA
OTANI, T. et al. "Determination of total and free
cholesterol by using cholesterol oxidase from
Streptomydes".**

(56) Entgegenhaltungen:
**CHEMICAL ABSTRACTS, vol. 89, Nr. 7,
14. August 1978, Seite 398,
Zusammenfassung 57 708d,
Columbus, Ohio, USA
IMSHENETSKII A. A. et al. "Effect of
cholesteroloxydase of Actinomyces lavendulae on
hypercholesterolemia in guinea pigs"**

**CHEMICAL ABSTRACTS, vol. 91, Nr. 3,
16. Juli 1979, Seite 238,
Zusammenfassung 15 711k
Columbus, Ohio, USA
PETROVA, L. Ya, et al. "Study of conditions for the
isolation of cholesterol oxidase from Actinomyces
lavendulae mycelium".**

**THE AMERICAN TYPE CULTURE COLLECTION,
Catalogue of Strains I,
14th Edition 1980
ATCC Rockville Maryland USA**

## Verfahren zur Gewinnung von Cholesterinoxidase

Die Erfindung betrifft ein Verfahren zur Gewinnung von Cholesterinoxidase aus Mikroorganismen.

Das Enzym Cholesterinoxidase hat grosse Bedeutung erlangt, da es eine sehr spezifische und empfindliche quantitative Bestimmung des Cholesterins, insbesondere in Körperflüssigkeiten, ermöglicht. Die Gewinnung des Enzyms erfolgt gewöhnlich aus Mikroorganismen, die auf einem Medium gezüchtet werden, welches einen oder mehrere Cholesterinoxidaseinduktoren enthält.

Trotz Zusatz von Induktoren sind jedoch die maximal erzielbaren Cholesterinoxidaseaktivitäten im Vergleich zu anderen Enzymen relativ gering, so dass ein Bedarf nach der Auffindung von Verfahren besteht, welche eine weitere Steigerung der gewinnbaren Literaktivitäten dieses Enzyms ermöglichen.

Als Induktor werden dabei solche Substanzen verwendet, welche selbst ein Substrat für das gewünschte Enzym darstellen oder mit einem derartigen Substrat chemisch nahe verwandt sind. Der Zusatz eines Cholesterinoxidaseinduktors ist erforderlich, um höhere Aktivitätsausbeuten zu erzielen, denn ohne das Vorhandensein eines Induktors ist es für Mikroorganismen völlig unökonomisch, ein Enzym zu bilden, für das es keine Verwendung gibt, wobei die Verwendung eben im enzymatischen Abbau der als Induktor fungierenden Substanz liegt. Ohne Induktor bilden verschiedene Mikroorganismen zwar ebenfalls Cholesterinoxidase, aber nicht in einem die Aufarbeitung lohnenden Mass, sondern in der Regel nur bis zu etwa 100 U/l.

Die Verwendung eines Cholesterinoxidaseinduktors erhöht zwar in der Regel die Aktivitätsausbeute an gesuchtem Enzym, sie kompliziert aber auch andererseits das Verfahren und beeinflusst die Reproduzierbarkeit negativ. Dies liegt darin begründet, dass die in Betracht kommenden Induktoren stark lipophilen Charakter aufweisen und daher nur schwierig in reproduzierbar feiner und gleichmässiger Verteilung dem wässrigen Kulturmedium der Mikroorganismen einverleibt werden können. Es wäre daher wünschenswert, ein Verfahren zu finden, welches eine Cholesterinoxidasegewinnung ohne Induktorzusatz bei der Züchtung in Ausbeuten ermöglicht, die mindestens denen entsprechen, die derzeit mit Verfahren unter Induktorzusatz erzielbar sind.

Der Erfindung liegt die Aufgabe zugrunde, die obigen Probleme ganz oder teilweise zu beseitigen. Insbesondere ist es Aufgabe der Erfindung, ein Verfahren zur Gewinnung von Cholesterinoxidase zu schaffen, welches es gestattet, höhere Enzymausbeuten als bei den bekannten Verfahren zu erzielen. Eine weitere Aufgabe besteht in der Auffindung eines derartigen Verfahrens, welches es ermöglicht, in Abwesenheit eines Cholesterinoxidaseinduktors zu arbeiten.

Gelöst wird diese Aufgabe erfindungsgemäss durch ein Verfahren zur Gewinnung von Cholesterinoxidase, welches dadurch gekennzeichnet ist, dass man Streptomyces griseofuscus DSM 40191, Streptomyces hygroscopicus DSM 40771, Streptomyces acidomyceticus DSM 40798 oder/und Arthrobacter paraffineus DSM 312 züchtet und das Enzym aus dem Kulturüberstand oder/und den Zellen gewinnt.

Mit den erfindungsgemäss verwendeten Mikroorganismen werden Aktivitäten erzielt, die weit über den bisher erreichbaren Werten liegen. Während bisher maximale Aktivitäten um 2000 U/l Kulturlösung erreicht wurden, gelingt es erfindungsgemäss, Aktivitäten bis zu etwa 6000 U/l Kulturlösung zu erzielen. Besonders überraschend ist jedoch, dass diese überlegenen Ausbeuten ohne Zusatz eines Cholesterinoxidaseinduktors erzielbar sind. Die erfindungsgemäss verwendeten Mikroorganismen stellen daher konstitutive Cholesterinoxidasebildner dar, welche gegenüber den besten bekannten konstitutiven Cholesterinoxidasebildnern eine bis zu 60-fach höhere Enzymaktivität liefern. Diese Aktivitätsangaben beziehen sich auf Fermentationsmedien mit einem Biomassegehalt von ca. 5 bis 10 g/l.

Die bei dem erfindungsgemässen Verfahren verwendeten Mikroorganismen geben das Enzym auch in den Kulturüberstand ab, wobei die Gesamtaktivität im Kulturüberstand in der Regel grösser ist als die Aktivität im Rohextrakt, der beim Aufschluss der Zellen gewonnen wird.

Die im erfindungsgemässen Verfahren verwendeten Mikroorganismen können auf den üblicherweise für Streptomyceten verwendeten Nährmedien unter aeroben Bedingungen, vorzugsweise in der Schüttelkultur, gezüchtet werden. Als besonders geeignet erwies sich ein Kulturmedium, welches 10 bis 30 g/l lösliche Stärke, 2 bis 10 g/l Pepton (Fleisch), 2 bis 10 g/l Hefeextrakt sowie die üblichen Salze, Spurenelemente und Vitamine enthält. Bei Verwendung eines derartigen Mediums erhält man maximale Ausbeuten in der Regel bei einer Züchtungsdauer zwischen 2 und 5 Tagen.

Die Erfindung ermöglicht es, durch Erzielung höherer Cholesterinoxidaseaktivitäten das Produktionsverfahren erheblich zu verbilligen. Von besonderem Vorteil ist dabei die Möglichkeit, dass ein Cholesterinoxidaseinduktor weggelassen werden kann, was nicht nur zu einer weiteren Verbilligung des Verfahrens, sondern auch zu einer erheblichen Steigerung der Reproduzierbarkeit desselben führt.

Die folgenden Beispiele erläutern die Erfindung weiter.

Beispiel 1

Streptomyces griseofuscus DSM 40191 (= ATCC 23916 = IFO 12870) wird in einem Medium folgender Zusammensetzung kultiviert:
20 g/l lösliche Stärke,

5 g/l Pepton (Fleisch),
4 g/l Hefeextrakt (Difco),
2 g/l $CaCO_3$,
1 g/l $KNO_3$,
0,5 g/l $K_2HPO_4$,
1,03 g/l $MgSO_4.7H_2O$,
0,5 g/l NaCl,
0,02 g/l $FeSO_4.7H_2O$.

Der Stamm wird zwei Tage in 10 ml Medium in einer 100 ml Erlenmeyer-Flasche kultiviert und dann in 40 ml des gleichen Mediums in 250 ml Erlenmeyerkolben übertragen. Anschliessend wird unter Schütteln bei 30°C gezüchtet. Nach 3 Tagen wird eine Probe entnommen und die Aktivität von Cholesterinoxidase im Kulturüberstand und im Rohextrakt geprüft. Der Rohextrakt wird erhalten durch Zentrifugieren von 5 ml Kulturlösung, Waschen der Biomasse mit 0,05 M Phosphat-Puffer, pH 7, Resuspendieren in 5 ml des gleichen Puffers und Aufschluss durch Zusatz von 0,2 ml 10%iger Triton-X100-Lösung. Nach 10 Minuten Stehen bei Raumtemperatur wurde zentrifugiert und der Überstand als Rohextrakt gewonnen.

Zur Durchführung der Aktivitätsbestimmung wurde die Cholestenonbildung anhand der Extinktionszunahme bei 240 nm gemessen, bei pH 7,5 in 0,5 Mol/l Phosphat-Puffer. Zur Kontrolle wurde jeweils ein Leerwert ohne Cholesterinzusatz bestimmt. Folgende Ergebnisse wurden erhalten:

Gesamt-U/l Kulturlösung:     5741
davon im Rohextrakt U/l:     1118
davon im Kulturüberstand U/l:     4623.

Beispiel 2
Das Verfahren von Beispiel 1 wurde wiederholt unter Verwendung von Streptomyces hygroscopicus DSM 40771 (= ATCC 10976).

Die Bestimmungen erfolgten nach 3 Tagen und 4 Tagen Kulturdauer. Folgende Aktivitäten wurden erhalten:

| Kulturdauer Tage | Gesamt- U/l | Rohex- trakt U/l | Kulturüber- stand U/l |
|---|---|---|---|
| 3 | 4765 | 1260 | 3505 |
| 4 | 5223 | 1423 | 3800 |

Wurde die Züchtung in Gegenwart von 2% Cholesterin als Induktor durchgeführt, so betrug die Aktivitätsausbeute knapp die Hälfte.

Beispiel 3
Das Verfahren von Beispiel 1 wurde wiederholt unter Verwendung von Streptomyces acidomyceticus DSM 40798 (= ATCC 11611 = IFO 3125). Die Ergebnisse bei einer Kulturdauer von 3 bzw. 4 Tagen zeigt die nachstehende Tabelle.

| Kulturdauer Tage | Gesamt- U/l | Rohex- trakt U/l | Kulturüber- stand U/l |
|---|---|---|---|
| 3 | 1656 | 721 | 935 |
| 4 | 2052 | 711 | 1341 |

Bei Durchführung der Züchtung in Gegenwart von 2% Cholesterin als Induktor stieg die Aktivitätsausbeute um 50%.

Beispiel 4
Das Verfahren von Beispiel 1 wurde wiederholt unter Verwendung von Arthrobacter paraffinens DSM 312 (= ATCC 15591), wobei dem Medium jedoch 3% Cholesterin in Form einer Suspension in Hefeextrakt (insgesamt 10 g/l) zugesetzt wurden. Nach 4 Tagen Kulturdauer wurden im Rohextrakt 4470 U/l gefunden. Auch im Kulturüberstand wurde eine beträchtliche Aktivität festgestellt.

**Patentansprüche**

1. Verfahren zur Gewinnung von Cholesterinoxidase, dadurch gekennzeichnet, dass man Streptomyces griseofuscus DSM 40191, Streptomyces hygroscopicus DSM 40771, Streptomyces acidomyceticus DSM 40798 oder/und Arthrobacter paraffineus DSM 312 züchtet und das Enzym aus dem Kulturüberstand oder/und den Zellen gewinnt.

2. Verfahren nach Anspruch 1, dadurch gekennzeichnet, dass man in einem Kulturmedium züchtet, welches keinen Cholesterinoxidaseinduktor enthält.

3. Verfahren nach Anspruch 1 oder 2, dadurch gekennzeichnet, dass man in einem Medium züchtet, welches 10 bis 30 g/l lösliche Stärke, 2 bis 10 g/l Pepton, 2 bis 10 g/l Hefeextrakt sowie Salze, Spurenelemente und Vitamine enthält.

**Patent Claims**

1. Process for obtaining of cholesterol oxidase, wherein Streptomyces griseofuscus DSM 40191, Streptomyces hygroscopicus 40771, Streptomyces acidomyceticus DSM 40798 and/or Arthobacter paraffinens DSM 312 are cultured and the enzyme is obtained from the culture supernatant and/or the cells.

2. Process according to claim 1, wherein culturing is carried out in a medium which does not contain a cholesterol oxidase inducer.

3. Process according to claim 1 or 2, wherein culturing is carried out in a medium which contains 10 to 30 g./litre of soluble starch, 2 to 10 g./litre of peptone and 2 to 10 g./litre yeast extract, as well as salts, trace elements and vitamins.

**Revendications**

1. Procédé pour obtenir de la cholestérol-oxydase, caractérisé en ce que l'on cultive Streptomyces griseofuscus DSM 40191, Streptomyces hygroscopicus DSM 40771, Streptomyces acidomyceticus DSM 40798 et/ou Arthrobacter paraffineus DSM 312 et on récupère l'enzyme à partir du liquide surnageant de la culture et/ou des cellules.

2. Procédé selon la revendication 1, caractérisé en ce que l'on cultive dans un milieu de culture qui ne contient pas d'inducteur de la cholestérol-oxydase.

3. Procédé selon la revendication 1 ou 2, caractérisé en ce que l'on cultive dans un milieu contenant 10 à 30 g/l d'amidon soluble, 2 à 10 g/l de peptone, 2 à 10 g/l d'extrait de levure ainsi que des sels, des obligo-éléments et des vitamines.